Europäisches Patentamt

European Patent Office

Office européen des brevets

(11) Veröffentlichungsnummer: **0 243 727 B1**

(12) **EUROPÄISCHE PATENTSCHRIFT**

(45) Veröffentlichungstag der Patentschrift: **14.10.92**

(21) Anmeldenummer: **87104962.3**

(22) Anmeldetag: **03.04.87**

(51) Int. Cl.⁵: **C12P 13/04**, C12P 13/06

(54) **Verfahren zur fermentativen Herstellung von L-Aminosäuren aus alpha-Ketocarbonsäuren.**

(30) Priorität: **02.05.86 DE 3614586**

(43) Veröffentlichungstag der Anmeldung:
**04.11.87 Patentblatt 87/45**

(45) Bekanntmachung des Hinweises auf die
Patenterteilung:
**14.10.92 Patentblatt 92/42**

(84) Benannte Vertragsstaaten:
**AT BE CH DE FR GB LI NL SE**

(56) Entgegenhaltungen:
**EP-A- 0 166 903**
**DE-A- 3 427 495**
**FR-A- 2 550 224**

(73) Patentinhaber: **Degussa Aktiengesellschaft**
**Weissfrauenstrasse 9**
**W-6000 Frankturt am Main 1(DE)**

Patentinhaber: **Forschungszentrum Jülich**
**GmbH**
**Postfach 1913**
**W-5170 Jülich(DE)**

(72) Erfinder: **Wandrey, Christian**
**Wolfshovener Strasse 139**
**W-5170 Jülich-Stetternich(DE)**
Erfinder: **Wichmann, Rolf**
**Talstrasse 2**
**W-5170 Jülich-Stetternich(DE)**
Erfinder: **Groeger, Ulrich**
**Siegfriedstrasse 70**
**W-4800 Bielefeld 1(DE)**
Erfinder: **Kircher, Manfred**
**Goldschmiedeweg 6**
**W-4800 Bielefeld 12(DE)**
Erfinder: **Leuchtenberger, Wolfgang**
**Geschwister-Scholl-Strasse 1**
**W-6454 Bruchköbel(DE)**
Erfinder: **Breuker, Eberhard**
**Rektenstrasse 10**
**W-4930 Detmold(DE)**

EP 0 243 727 B1

**Beschreibung**

Die Erfindung bezieht sich auf ein Verfahren zur Herstellung von L-Aminosäuren aus α-Ketocarbonsäuren durch kontinuierliche Fermentation mit Hilfe von glutamatproduzierenden Bakterien in Gegenwart von Ammoniumionen und eines Energielieferanten, insbesondere von Glucose, unter Biomasserückhaltung.

L-Aminosäuren können aus α-Ketocarbonsäuren durch enzymatische Umwandlung erhalten werden. Auch die mikrobielle Umwandlung ist bereits bekannt: So wird in der DE-OS 34 27 495 ein Verfahren beschrieben, bei dem glutamatausscheidende Bakterien, insbesondere der Gattungen Brevibacterium und Corynebacterium, innerhalb von 6 - 40 Stunden in einem Nährmedium angezüchtet werden, wonach die umzusetzende α-Ketocarbonsäure speziell während der logarithmischen Wachstumsphase (zwischen etwa 20 und 72 Stunden) zur Kultur hinzugegeben und in L-Aminosäure umgewandelt wird.

Nach einem weiteren, von den Anmeldern beschriebenen Verfahren (DE-Patentanmeldung 34 19 585.8) werden ebenfalls L-Aminosäuren durch mikrobielle Umwandlung von α-Ketocarbonsäuren mit Hilfe von glutamatproduzierenden Bakterien der genannten Art unter Verwendung von wachsenden oder nichtwachsenden Zellen unter aeroben Bedingungen zwischen 20 und 45 °C und pH 5 - 9 innerhalb von 1 - 10 Tagen erhalten. Dieses Verfahren kann, wie angegeben wird, kontinuierlich unter Biomasserückhaltung durchgeführt werden.

Die genannten mikrobiellen Verfahren sind relativ zeitaufwendig, und es ist daher die Aufgabe der vorliegenden Erfindung, die Wirtschaftlichkeit der mikrobiellen Umwandlung von α-Ketocarbonsäuren in L-Aminosäuren zu verbessern.

Diese Aufgabe wird durch das erfindungsgemäße Verfahren gelöst, daß im wesentlichen dadurch gekennzeichnet ist, daß mit einer Zellkonzentration im Fermenter von ≥ 30 g TS/l, insbesondere ≥ 60 g TS/l, Verweilzeiten von 1 - 20 Stunden, insbesondere 6 - 10 Stunden, und mit einer solchen Belüftung und Luftdispergierung gearbeitet wird, daß die L-Lactatkonzentration der Produktlösung unter 40 mMol/l bleibt.

Weitere Besonderheiten gehen aus den Unteransprüchen hervor.

Beim erfindungsgemäßen Verfahren wird mit besonders hohen Zellkonzentrationen und kurzen Verweilzeiten im Durchflußfermenter gearbeitet, wobei gleichzeitig eine Nährstoff- und insbesondere Sauerstoff-Limitierung vermieden wird.

Während bei kontinuierlichen Verfahren mit Biomasserückhaltung üblicherweise eine Teilabführung von Zellmaterial mit dem Produktstrom erfolgt, um die Anteile an alten Zellen und Zellbruchstücken auf ein begrenztes Maß zu reduzieren und eine gewünschte Altersverteilung zu erhalten, wird gemäß der Erfindung eine sehr weit getriebene Akkumulation von Zellen vorgesehen, die praktisch vollständig im Fermenter zurückgehalten werden.

Mit der Akkumulation der Zellmasse im Fermenter durch Filtration und/oder Separation ist selbstverständlich eine ausreichend rasche Rückführung der Zellmasse in den Fermenter verbunden, die durch einen Mindestvolumenstrom im externen Kreislauf erreicht wird, der technische Begrenzungen wie Druckabfall, Schaumbildung usw. und den Sauerstoffbedarf der Zellen berücksichtigt.

Durch diese weitgehende Rückhaltung der Zellen im Fermenter erhält man eine praktisch zellfreie Produktlösung und eine so hohe Zellkonzentration im Fermenter, daß dadurch eine Wachstumsbegrenzung der Bakterien gegeben ist, wobei jedoch eine Sauerstoff- oder Nährstoff-Limitierung vermieden wird.

Unter diesen Bedingungen wird eine hohe Selektivität der L-Aminosäurebildung erreicht bei kurzer Verweilzeit und hohem Umsatz; d.h. hohen Raumzeitausbeuten.

Es ergibt sich daraus sowohl ein wirtschaftlicher Betrieb des Fermenters als auch eine Vereinfachung der Aufarbeitung der Produktlösung, wozu zusätzlich beitragen kann, daß gemäß einer bevorzugten Ausführungsart ohne Zufuhr von Komplexmedium (wie Hefeextrakt, Fleischextrakt, Proteinhydrolysat usw.) gearbeitet wird.

Im übrigen entsprechen die Zusammensetzung des Kulturmediums, sein pH-Wert und die Temperatur der bekannten Verfahrensweise, wobei allerdings vorzugsweise ohne Komplexmedium gearbeitet wird. Als Energielieferant dient insbesondere wie üblich Glucose. Alternativ können selbstverständlich andere Energielieferanten, wie Zucker allgemein, Acetat usw. vorgesehen werden. Die Anwesenheit dieses Energielieferanten ist notwendig, um für die Produktion der L-Aminosäure ausreichende Mengen an Reduktionsäquivalenten bereitzustellen und den Erhaltungsstoffwechsel zu gewährleisten. Bei kurzer Verweilzeit und begrenztem Zellwachstum kann der Energielieferant nicht mehr vollständig genutzt werden, wobei dann die Verwertung von gebildeter L-Aminosäure durch die Bakterien auf jeden Fall vernachlässigbar ist.

Die ausreichende Sauerstoffversorgung ist über die L-Lactatkonzentration in der Produktlösung definiert: Bekannterweise wird L-Lactat von den Bakterien ausgeschieden, wenn die Versorgung derselben mit Sauerstoff limitiert ist. Durch Messung der L-Lactatkonzentration im Produktstrom kann somit auf eine ausreichende oder nichtausreichende Sauerstoffversorgung der Bakterien geschlossen werden.

Die folgende Tabelle gibt Beispiele für den erfindungsgemäßen Arbeitsbereich:

| Betriebspunkt | 1 | 2 | 3 |
|---|---|---|---|
| Zelltrockenmasse $\left[\frac{g \ TS}{l}\right]$ | 32,4 | 82,2 | 95,3 |
| Verweilzeit [h] | 19,6 | 8,1 | 3,1 |
| Raum-Zeit-Ausbeute $\left[\frac{g}{l \cdot d}\right]$ | 16,4 | 38,3 | 79,4 |
| Umsatz [%] | 98,1 | 94,2 | 81,9 |
| Selektivität | 0,852 | 0,892 | 0,902 |
| Ausbeute | 0,836 | 0,840 | 0,738 |

In dem beigefügten Kurvenbild sind die Selektivität und die Ausbeute an L-Aminosäure über den erreichten Ketosäureumsätzen dargestellt.

Die Versuchsdaten beziehen sich auf die Erzeugung von L-Leucin aus α-Ketoisocaproat mit Hilfe von Corynebacterium glutamicum bei pH 7,2; 30 °C; Luftdurchsatz 0,16 VVm; Drehzahl 600 Upm;

$$P_{O_2} \sim 20 \ \%$$

und Redoxspannung:~0 mV.

Wie aus den Versuchsdaten hervorgeht, können mit Verweilzeiten zwischen etwa 6 und 10 Stunden und Zellkonzentrationen im Fermenter um 90 g TS/l Umsätze über 90 % erzielt werden, bei denen, wie aus dem Kurvenbild hervorgeht, die Selektivität noch keinen merklichen Abfall zeigt. Dabei erhält man Raumzeitausbeuten über 50 g/l/d.

Nachfolgend werden Beispiele für die erfindungsgemäße Erzeugung von L-Leucin und L-Isoleucin beschrieben.

Beispiel 1

Gewinnung von L-Leucin aus α-Ketoisocaproat und Ammonium mit Corynebacterium glutamicum (ATCC 13032) in einer kontinuierlich betriebenen Fermentation mit Zellrückhaltung durch Filtration.

Durch Einsatz zweier, parallel geschalteter Mikrofiltrationsrohrbündel-Module der Firma Enka, Typ "HA 11 24 H 32" konnte bei konstantem Füllvolumen des Fermenters Produktlösung zellfrei abgepumpt werden. Diese Filtermodule haben eine Membranfläche von 0,05 Quadratmeter und eine Porengröße von 300 nm.

Das Produktionsmedium enthielt: 40 g/l Glucose, 30 g/l Ammoniumsulfat, 20 g/l Na-α-Ketoisocaproat, 1 g/l Kaliumhydrogenphosphat, 0,25 g/l Magnesiumsulfat-Heptahydrat, 10 mg/l Calciumchlorid-Dihydrat, 10 mg/l Eisensulfat-Heptahydrat, 7,6 mg/l Mangansulfat-Tetrahydrat, 0,4 mg/l Biotin und 1 ml/l Polypropylenglykol P1200 (Fa. Roth). Der pH-Wert wurde mit Natronlauge auf 7,2 eingestellt.

Als Vorvorkultur wurde in einem 500 ml Erlenmeyerkolben mit 2 Schikanen 100 ml einer Nährlösung mit Zellen von Corynebakterium glutamicum (ATCC 13032) beimpft. Die Nährlösung enthielt 20 g/l Glucose, 10 g/l Bacto-Pepton (Difco), 10 g/l Hefeextrakt (Merck) und 2,5 g/l Natriumchlorid. Der pH-Wert wurde mit Natronlauge auf 7,2 eingestellt. Nach 24 Stunden schütteln mit 100 UpM bei 30 Grad Celsius wurde die erhaltene Zellsuspension in einen 2 l Erlenmeyerkolben mit 2 Schikanen mit 500 ml einer Nährlösung

3

gegeben. Diese Nährlösung hatte die gleiche Zusammensetzung wie das Produktionsmedium, nur wurden 20 g/l Calciumcarbonat als Puffersubstanz verwendet. Calciumchlorid wurde nicht verwendet. Der pH-Wert dieser Nährlösung wurde vor Zugabe des Calciumcarbonats mit Natronlauge auf 7,2 eingestellt. Nach 24 Stunden schütteln mit 100 UpM bei 30 Grad Celsius wurde die erhaltene Zellsuspension als Inokulum zu 3 l Produktionsmedium in den Fermenter gegeben.

Die Nährlösungen wurden vor der Verwendung 30 Minuten bei 121 Grad Celsius und 1,1 Bar Dampfdruck autoklaviert. Es wurde Glucose, α-Ketoisocaproat und die restlichen Bestandteile bis auf Calciumcarbonat jeweils getrennt autoklaviert. Calciumcarbonat wurde trocken 8 Stunden bei 150 Grad Celsius sterilisiert. Nach dem Erkalten wurden alle Nährmedienbestandteile steril vereinigt. Der Fermenter und das Mikrofiltrationsmodul wurden mit Wasser 30 Minuten bei 121 Grad Celsius und 1,1 Bar Dampfdruck sterilisiert. Nach der Sterilisation wurde das zur feuchten Sterilisation verwendete Wasser aus dem Fermenter und dem Mikrofiltrationsmodul steril abgelassen und es wurde der Fermenter mit 3 l Produktionsmedium steril befüllt.

1,8 Tage nach Animpfung des Fermenters, nachdem die im Nährmedium vorhandene Glucose verbraucht war und ca. 7 g-Zelltrockenmasse/l an Bakterien gewachsen waren, wurde begonnen steril Produktionsmedium in den Fermenter zu pumpen. Gleichzeitig wurde der gleiche Volumenstrom an Produktlösung zellfrei aus dem Fermenter gepumpt. Zur Abtrennung der Bakterien aus der Produktlösung wurde von diesem Zeitpunkt an der Inhalt des Fermenters ständig mit einer Zahnradpumpe mit 250 l/h im Kreislauf durch das Innere des Hohlfaser-Filtrationsmodul gepumpt. Durch diese Strömung sollte die Bildung eines Belags an Zellen auf der Filtrationsoberfläche möglichst gering gehalten werden.

3,5 Tage wurde die Fermentation mit einer Verweilzeit von 7,2 Stunden, dann 29 Tage mit einer Verweilzeit von 8,1 Stunden betrieben. Die Konzentration an Bakterien wurde durch periodische Entfernung von zellhaltiger Lösung bei ca. 35 g-Zelltrockenmasse konstant gehalten. Die Zellwachstum betrug durchschnittlich 6 %/Tag.

Während des kontinuierlichen Betriebs der Fermentation lag der Umsatz zwischen 85 und 95 % bei über 95 % Selektivität. Es wurden bis zu 15,5 g-L-Leucin/l produziert.

2. Beispiel

Gewinnung von L-Leucin aus α-Ketoisocaproat und Ammonium mit Corynebacterium glutamicum (ATCC 13032) in einer kontinuierlich betriebenen Fermentation mit Zellrückhaltung durch Filtration.

Durch Einsatz eines Mikrofiltrationsrohrbündel-Moduls der Firma Enka, Typ "MD 080 TP 2N" wurde 5,8 Tage bei konstantem Füllvolumen des Fermenters Produktlösung zellfrei abgepumpt werden. Die Filtrationsmembranfläche des Enka-Moduls betrug 1 Quadratmeter, die Porengröße betrug 200 nm. Zur Förderung der Zellsuspension im Kreislauf durch das Enka-Modul diente eine Zahnradpumpe, der Kreislaufvolumenstrom betrug ca. 300 l/h.

Vor Beginn der Fermentation wurde der Fermenter, die Filtrationsanlage und die Nährmedien wie in Beispiel 1 beschrieben sterilisiert und mit Nährmedium befüllt. Die Zusammensetzung dieses Produktionsmediums, sowie weiterer 75 l für den kontinuierlichen Betrieb des Fermenters und das Medium der 2. und der 3. Vorkultur unterschied sich vom vorher beschriebenen Produktionsmedium dadurch, daß 6% Glucose und 4% Ammoniumsulfat anstatt 4% bzw. 3% verwendet wurden. Die restliche Zusammensetzung war gleich der auch sonst verwendeten. Die 2. und 3. Vorkultur enthielten 2% Calciumcarbonat.

Als 1. Vorkultur wurden 100 ml Vollmedium mit einem pH 7,4 und zusätzlich 0,025% Magnesiumsulfat, neben den in Beispiel 1 beschriebenen Komponenten, mit Corynebacterium Glutamicum (ATCC 13032) beimpft und 31 Stunden bei 30 Grad Celsius und 100 UpM geschüttelt. Dann wurden 2 ml Zellsuspension als Animpfmenge in einen Kolben mit 98 ml Produktionsmedium überführt. Diese 2. Vorkultur wurde 89 Stunden bei 30 Grad Celsius und 100 UpM geschüttelt. Wiederum wurden 2 ml Zellsuspension als Animpfmenge in einen Kolben mit 98 ml Produktionsmedium überführt. Diese 3. Vorkultur wurde 55 Stunden bei 30 Grad Celsius und 100 UpM geschüttelt und dann komplett zum Animpfen des Fermenters verwendet.

Nachdem die Anzuchtphase beendet war und die Glucose verbraucht war wurde wie in Beipiel 1 beschrieben der Fermenter in kontinuierlicher Betriebsweise betrieben. 5,8 Tage lang wurde das Hohlfaser-Filtermodul zur Zellrückhaltung verwendet. Bei einer Verweilzeit von 19,6 Stunden wurde ein Umsatz von 98,1 % erreicht, bei einer Selektivität von 85,2 %, die L-Leucin-Konzentration betrug 13,4 g/l. Bei einer Verweilzeit von 3,1 Stunden betrug der Umsatz 81,9 % bei 90,2 % Selektivität. Die L-Leucin-Konzentration betrug 10,4 g/l bei einer Raum-Zeit-Ausbeute von 79,4 g-L-Leucin pro l-Reaktionsvolumen und Tag. Die Konzentration an Bakterien betrug bei 19,6 Stunden Verweilzeit ca. 32 g-Zelltrockenmasse pro l, bei 3,1 Stunden Verweilzeit ca. 95 g-Zelltrockenmasse pro l.

3. Beispiel

Gewinnung von L-Isoleucin aus $\alpha$-Ketobutyrat und Ammonium mit Corynebacterium glutamicum (ATCC 13032) in einer kontinuierlich betriebenen Fermentation mit Zellrückhaltung durch Filtration.

Durch Einsatz eines Mikrofiltrationsrohrbündel-Moduls der Firma Enka, Typ "MD 080 TP 2N" wurde konnte bei konstantem Füllvolumen des Fermenters Produktlösung zellfrei abgepumpt werden. Die Filtrationsmembranfläche des Enka-Moduls betrug 1 Quadratmeter, die Porengröße betrug 200 nm. Zur Förderung der Zellsuspension im Kreislauf durch das Enka-Modul diente eine Zahnradpumpe, der Kreislaufvolumenstrom betrug ca. 300 l/h.

Vor Beginn der Fermentation wurde der Fermenter, die Filtrationsgeräte und die Nährmedien wie in Beispiel 1 beschrieben sterilisiert und mit Nährmedium befüllt.

Das Produktionsmedium enthielt: 40 g/l Glucose, 20 g/l Ammoniumsulfat, 20 g/l Na-$\alpha$-Ketobutyrat, 1 g/l Kaliumhydrogenphosphat, 1 g/l Magnesiumsulfat-Heptahydrat, 10 mg/l Calciumchlorid-Dihydrat, 10 mg/l Eisensulfat-Heptahydrat, 7,6 mg/l Mangansulfat-Tetrahydrat, 0,4 mg/l Biotin 0,1 g/l L-Valin, 0,1 g/l L-Leucin und 1 ml/l Polypropylenglykol P1200 (Fa. Roth). Der pH-Wert wurde mit Natronlauge auf 7,4 eingestellt.

Als 1. Vorkultur wurden 100 ml Vollmedium mit einem pH 7,4 und zusätzlich 0,025% Magnesiumsulfat, neben den in Beispiel 1 beschriebenen Komponenten, mit Corynebacterium Glutamicum (ATCC 13032) beimpft und 23 Stunden bei 30 Grad Celsius und 100 UpM geschüttelt. Dann wurden 60 ml Zellsuspension als Animpfmenge in einen Kolben mit 600 ml Produktionsmedium gegeben. Dieses Produktionsmedium enthielt 20 g/l Glucose, 10 g/l Na-$\alpha$-Ketobutyrat und 20 g/l Calciumcarbonat anstatt bzw. zusätzlich zu den oben angegebenen Komponenten.

Diese 2. Vorkultur wurde 27 Stunden bei 30 Grad Celsius und 100 UpM geschüttelt und dann komplett zum Animpfen des Fermenters verwendet.

Nachdem die Anzuchtphase beendet war und die Glucose verbraucht war wurde wie in Beipiel 1 beschrieben der Fermenter in kontinuierlicher Betriebsweise betrieben. 11 Tage lang wurde das Hohlfaser-Filtermodul zur Zellrückhaltung verwendet. Nach 4 Tragen kontinuierlichem Betrieb der Fermentation wurde die Glucose-Konzentration im Nährmedium auf 60 g/l angehoben. Während der Fermentation wurde der pH-Wert durch Dosierung von Natronlauge bei 7,4 konstant gehalten. Durch eine ausreichende Belüftung konnte eine Sauerstofflimitierung vermieden werden, so keine nennenswerte Menge an Lactat im der Produktlösung gemessen werde.

Bei einer Verweilzeit von 7 Stunden wurde ein Umsatz von 66 % erreicht, bei einer Selektivität von 54 %, die L-Iso-Leucin-Konzentrationbetrug 5,5 g/l, bei einer Raum-Zeit-Ausbeute von 17,5 g-L-Isoleucin pro l-Reaktionsvolumen und Tag. Die Konzentration an Bakterien betrug ca. 92 g-Zelltrockenmasse pro l.

4. Beispiel

Gewinnung von L-Isoleucin aus $\alpha$-Ketobutyrat und Ammonium mit Brevibacterium flavum Variante DM40-3 (DSM 3812) abgeleitet von ATCC 14067) in einer kontinuierlich betriebenen Fermentation mit Zellrückhaltung durch Filtration.

Durch Einsatz eines Mikrofiltrationsrohrbündel-Moduls der Firma Enka, Typ "MD 080 TP 2N" konnte bei konstantem Füllvolumen des Fermenters Produktlösung zellfrei abgepumpt werden. Die Filtrationsmembranfläche des Enka-Moduls betrug 1 Quadratmeter, die Porengröße betrug 200 nm. Zur Förderung der Zellsuspension im Kreislauf durch das Enka-Modul diente eine Zahnradpumpe, der Kreislaufvolumenstrom betrug ca. 300 l/h.

Vor Beginn der Fermentation wurde der Fermenter, die Filtrationsgeräte und ein Nährmedienbehälter wie in Beispiel 1 beschrieben sterilisiert. Die Nährmedienbestandteile wurden mit Ausnahme der Spurenelemente steril filtriert zugegeben. Die Spurenelemente waren bereits im zur Sterilisierung des Nährmedienbehälters verwendeten Wasser enthalten.

Das Produktionsmedium enthielt: 100 g/l Glucose, 20 g/l Ammoniumsulfat, 8 g/l $\alpha$-Ketobuttersäure, 1 g/l Kaliumhydrogenphosphat, 1 g/l Magnesiumsulfat-Heptahydrat, 10 mg/l Calciumchlorid-Dihydrat, 10 mg/l Eisensulfat-Heptahydrat, 5 mg/l Mangansulfat-Tetrahydrat, 1 mg/l Biotin und 1 ml/l Polypropylenglykol P1200 (Fa. Roth). Der pH-Wert wurde mit Natronlauge auf 7,4 eingestellt.

Als 1. Vorkultur wurden 100 ml eines Mediums mit 30 g/l "Caso-Boullion" (Merck), das mit Natronlauge auf pH 7,4 eingestellt wurde, mit Brevibacterium flavum Variante DM40-3 (abgeleitet von ATCC 14067) beimpft und 16 Stunden bei 30 Grad Celsius und 100 UpM geschüttelt. Dann wurden 60 ml Zellsuspension als Animpfmenge in einen Kolben mit 600 ml vom gleichen Medium gegeben.

Diese 2. Vorkultur wurde 4 Stunden bei 30 Grad Celsius und 100 UpM geschüttelt und dann komplett zum Animpfen des Fermenters verwendet.

Nachdem die Anzuchtphase beendet war und die Glucose verbraucht war wurde wie in Beipiel 1 beschrieben der Fermenter in kontinuierlicher Betriebsweise betrieben. 18 Tage lang wurde das Hohlfaser-Filtermodul zur Zellrückhaltung verwendet. Während der Fermentation wurde der pH-Wert durch Dosierung von Natronlauge bei 7,4 konstant gehalten.

Bei einer Verweilzeit von 5 Stunden wurde eine L-Isoleucin-Konzentration betrug 2,5 g/l, bei einer Raum-Zeit-Ausbeute von 13,5 g-L-Isoleucin pro l-Reaktionsvolumen und Tag. Die Konzentration an Bakterien betrug ca. 80 g-Zelltrockenmasse pro l.

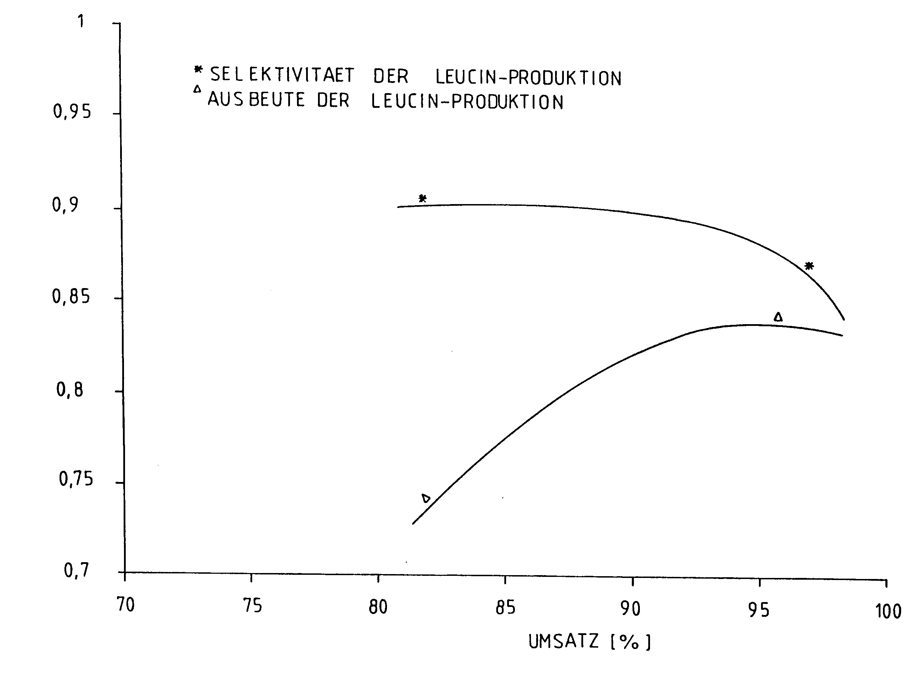

**Patentansprüche**

1.  Verfahren zur Herstellung von L-Aminosäuren aus $\alpha$-Ketocarbonsäuren durch kontinuierliche Fermentation mit Hilfe von glutamatproduzierenden Bakterien in Gegenwart von Ammoniumionen und eines Energielieferanten, insbesondere von Glucose, unter Biomasserückhaltung und pH-Kontrolle dadurch gekennzeichnet, daß mit einer Zellkonzentration im Fermenter von $\geq$ 30 g TS/l, insbesondere $\geq$ 60 g TS/l, Verweilzeiten von 1 bis 20 Stunden, insbesondere 6 bis 10 Stunden, gearbeitet und die Belüftung und Luftdispergierung über die in der Produktlösung vorliegende L-Lactatkonzentration derart gesteuert wird, daß letztere unter 40 mMol/l bleibt.

2.  Verfahren nach Anspruch 1,
    **dadurch gekennzeichnet,**
    daß die Zellkonzentration im Fermenter bei 80 bis 120 g TS/l, insbesondere um 95 g TS/l, liegt.

3.  Verfahren nach Anspruch 1 oder 2,
    **dadurch gekennzeichnet,**
    daß ohne Zufuhr von Komplexmedium gearbeitet wird.

4.  Verfahren nach einem der Ansprüche 1 bis 3,
    **dadurch gekennzeichnet,**
    daß die Biomesserückhaltung durch Mikrofiltration durchgeführt wird.

5.  Verfahren nach Anspruch 4,
    **dadurch gekennzeichnet,**
    daß Hohlfaserfilter für die Mikrofiltration verwendet werden.

6.  Verfahren nach Anspruch 4 oder 5,
    **dadurch gekennzeichnet,**
    daß ein Filter mit einem mittleren Porendurchmesser von maximal 300 nm verwendet wird.

7.  Verfahren nach einem der Ansprüche 1 bis 3,
    **dadurch gekennzeichnet,**
    daß für die Bakterienrückhaltung ein Zentrifugal-Tellerseparator verwendet wird mit absatzweisem oder stetigem Austrag von Zellen, die insgesamt in den Fermenter zurückgeführt werden.

8.  Verfahren nach Anspruch 7,
    **dadurch gekennzeichnet,**
    daß bei absatzweisem Zellaustrag mit Zeitabständen von 1 bis 20 min für die Totalentleerung bei selbstklärenden Tellerseparatoren gearbeitet wird.

9.  Verfahren nach Anspruch 7 oder 8, gekennzeichnet durch eine Mikrofiltration des zellarmen Ablaufs der Zentrifugalseparation.

10. Verfahren nach einem der vorangehenden Ansprüche,
    **dadurch gekennzeichnet,**
    daß als Bacterium ein Vertreter der Gattung Corynebacterium oder Brevibacterium, insbesondere ein Stamm von Corynebacterium glutamicum oder Brevibacterium flavum verwendet wird.

11. Verfahren nach Anspruch 10,
    **dadurch gekennzeichnet,**

daß man Corynebacterium glutamicum ATCC 13032 oder Brevibacterium flavum Variante DM40-3 (DSM 3812) (abgeleitet von ATCC 14067) verwendet wird.

## Claims

1. A process for the production of L-amino acids from $\alpha$-ketocarboxylic acids by continuous fermentation using glutamate-producing bacteria in the presence of ammonium ions and an energy source, more particularly glucose, with retention of biomass and pH control, characterized in that it is carried out with a cell concentration in the fermenter of $\geq$ 30 g dry matter/l and, more particularly, $\geq$ 60 g dry matter/l and with residence times of 1 to 20 hours and, more particularly, 6 to 10 hours and in that aeration and air dispersion are controlled through the L-lactate concentration in the product solution in such a way that the L-lactate concentration remains below 40 mmol/l.

2. A process as claimed in claim 1, characterized in that the cell concentration in the fermenter is between 80 and 120 g dry matter/l and, more particularly, around 95 g dry matter/l.

3. A process as claimed in claim 1 or 2, characterized in that it is carried out without introduction of complex medium.

4. A process as claimed in any of claims 1 to 3, characterized in that the biomass is retained by microfiltration.

5. A process as claimed in claim 4, characterized in that hollow fiber filters are used for the microfiltration.

6. A process as claimed in claim 4 or 5, characterized in that a filter having a mean pore diameter of at most 300 nm is used.

7. A process as claimed in any of claims 1 to 3, characterized in that a centrifugal disk separator is used for bacteria retention with batch or continuous discharge of cells which are all returned to the fermenter.

8. A process as claimed in claim 7, characterized in that, where the cells are removed in batches, time intervals of 1 to 20 mins. are allowed for complete emptying in the case of self-clarifying disk separators.

9. A process as claimed in claim 7 or 8, characterized by microfiltration of the low-cell effluent of the centrifugal separator.

10. A process as claimed in any of the preceding claims, characterized in that a representative of the genus Corynebacterium or Brevibacterium, more particularly a strain of Corynebacterium glutamicum or Brevibacterium flavum, is used as the bacterium.

11. A process as claimed in claim 10, characterized in that Corynebacterium glutamicum ATCC 13032 or Brevibacterium flavum variant DM40-3 (DSM 3812) (derived from ATCC 14067) is used.

## Revendications

1. Procédé pour la production de L-aminoacides à partir d'acides $\alpha$-cétocarboxyliques par fermentation continue à l'aide de bactéries productrices de glutamate, en présence d'ions ammonium et d'un générateur d'énergie, en particulier de glucose, avec rétention de biomasse et régulation du pH, caractérisé en ce que l'on opère avec une concentration de cellules dans le fermenteur de $\geq$ 30 g/de matière sèche (MS) par litre, en particulier de $\geq$ 60 g MS/l, des temps de séjour de 1 à 20 heures, en particulier de 6 à 10 heures, et on règle l'aération et la dispersion d'air, par l'intermédiaire de la concentration de L-lactacte présente dans la solution de produit, de manière que cette dernière reste inférieure à 40 m.moles/l.

2. Procédé selon la revendication 1, caractérisé en ce que la concentration de cellules dans le fermenteur est de 80 à 120 g MS/l, en particulier d'environ 95 g MS/l.

3. Procédé selon la revendication 1 ou 2, caractérisé en ce que l'on opère sans apport de milieu complexe.

4. Procédé selon l'une quelconque des revendications 1 à 3, caractérisé en ce que l'on effectue la rétention de biomasse par microfiltration.

5. Procédé selon la revendication 4, caractérisé en ce que l'on utilise pour la microfiltration des filtres à fibres creuses.

6. Procédé selon la revendication 4 ou 5, caractérisé en ce que l'on utilise un filtre ayant un diamètre de pores moyen de 300 nm au maximum.

7. Procédé selon l'une quelconque des revendications 1 à 3, caractérisé en ce que l'on utilise pour la rétention de bactéries un séparateur à disque centrifuge avec extraction statique ou discontinue de cellules, qui sont recyclées en totalité dans le fermenteur.

8. Procédé selon la revendication 7, caractérisé en ce que, dans le cas d'extraction discontinue de cellules, on opère avec des intervalles de temps de 1 à 20 minutes pour le vidage total dans des séparateurs à disque autonettoyants.

9. Procédé selon la revendication 7 ou 8, caractérisé par une microfiltration de l'effluent pauvre en cellules de la séparation centrifuge.

10. Procédé selon l'une quelconque des revendications précédentes, caractérisé en ce que l'on utilise en tant que bactérie un représentant du genre Corynebacterium ou Brevibacterium, en particulier une souche de Corynebacterium glutamicum ou de Brevibacterium flavum.

11. Procédé selon la revendication 10, caractérisé en ce que l'on utilise Corynebactérium glutamicum ATCC 13032 ou Brevibacterium flavum, variante DM40-3 (DSM 3812) (dérivé de ATCC 14067).